# EUROPEAN PATENT APPLICATION

(11) **EP 4 585 134 A1**
(43) Date of publication of application: **16.07.2025**
(21) Application number: 25151047.5
(22) Date of filing: 09.01.2025
(51) Int. Cl.: A61B 1/00, A61B 1/267, A61B 1/273

(54) **IMAGE PROCESSING DEVICE, OPERATION METHOD OF IMAGE PROCESSING DEVICE, NON-TRANSITORY COMPUTER READABLE MEDIUM, AND DIAGNOSIS SUPPORT APPARATUS**

(30) Priority: 12.01.2024 JP 2024003023
(71) Applicant: FUJIFILM Corporation, Tokyo 106-8620 (JP); Fujita Academy, Toyoake-shi, Aichi 470-1192 (JP)
(72) Inventor: TERAMURA, Yuichi, Kaisei-machi, Ashigarakami-gun, Kanagawa (JP); OTAKA, Yohei, Toyoake, Aichi 470-1192 (JP); SHIBATA, Seiko, Toyoake, Aichi 470-1192 (JP)
(74) Representative: HGF

(57) **Abstract**

Provided are an image processing device, an operation method of an image processing device, an image processing program, and a diagnosis support apparatus capable of easily obtaining an objective and highly accurate indicator related to swallowing without taking time and effort.

An image processing device acquires an examination video in which an observation target is imaged in a video endoscopic examination of swallowing, performs recognition processing on the examination video for each frame image, to assign stage information indicating that an image shows which stage of the observation target among a plurality of swallowing stages set in advance to each of the frame images, and calculates a feature value indicating a feature related to swallowing of a subj ect person having the observation target based on the stage information assigned to each of a plurality of the frame images included in the examination video.

## Description

### BACKGROUND OF THE INVENTION

### 1. Field of the Invention

The present invention relates to an image processing device, an operation method of an image processing device, a non-transitory computer readable medium, and a diagnosis support apparatus.

### 2. Description of the Related Art

Since dysphagia occurs in association with aging or a nervous system disease, the importance of an examination on a swallowing function has been increasing in recent years in an aging society. The examination on the swallowing function is performed to specify a pathological condition of aspiration and to appropriately perform treatment or prevention of dysphagia. A video endoscopic examination of swallowing (VE) or a fiberoptic endoscopic evaluation of swallowing (FEES) has been established as an evaluation method for dysphagia (swallowing function evaluation examination).

Various devices for performing evaluation and the like based on the acquired examination image in a video endoscopic examination of swallowing are known (JP2022-179218A or JP2022-179222A).

### SUMMARY OF THE INVENTION

In the video endoscopic examination of swallowing in the related art, a doctor observes an image of a swallowing movement obtained through an endoscope to perform an evaluation, a diagnosis, and the like. That is, the diagnosis is mainly performed by a subjective evaluation of the doctor.

However, there is a problem in that, in a case of the diagnosis made only by a subjective evaluation by a doctor's visual examination, it is difficult to make a diagnosis with higher accuracy because the diagnosis result varies depending on a skill of the doctor and the like.

An object of the present invention is to provide an image processing device, an operation method of an image processing device, a non-transitory computer readable medium, and a diagnosis support apparatus capable of obtaining an objective and highly accurate indicator related to swallowing without taking time and effort.

An aspect of the present invention relates to an image processing device comprising: a control processor, in which the control processor acquires an examination video in which an observation target is imaged in a video endoscopic examination of swallowing, performs recognition processing on the examination video for each frame image, to assign stage information indicating that an image shows which stage of the observation target among a plurality of swallowing stages set in advance to each of the frame images, and calculates a feature value indicating a feature related to swallowing of a subject person having the observation target based on the stage information assigned to each of a plurality of the frame images included in the examination video.

It is preferable that the stage information includes a swallowing-in-progress stage indicating that the observation target is swallowing and a swallowing-not-in-progress stage indicating that the observation target is not swallowing, and the swallowing-in-progress stage includes an initial swallowing-in-progress stage indicating that the observation target is in an initial stage of the swallowing that is in progress and a late swallowing-in-progress stage indicating that the observation target is in a late stage of the swallowing that is in progress.

It is preferable that the control processor detects a swallowing block that is a group of the frame images in which one swallowing motion of the observation target is imaged, and the swallowing block includes a plurality of consecutive frame images in which the stage information is the swallowing-in-progress stage.

It is preferable that the feature value is the number of the swallowing blocks.

It is preferable that the feature value is a basic statistic based on the number of the frame images included in each of the swallowing blocks.

It is preferable that the feature value is the number of the frame images in which the stage information is the initial swallowing-in-progress stage.

It is preferable that the feature value is a basic statistic based on the number of the frame images in which the stage information is the initial swallowing-in-progress stage in the frame images included in the swallowing block.

It is preferable that the swallowing-not-in-progress stage includes a post-swallowing stage in which the stage information of an immediately preceding frame image is the late swallowing-in-progress stage, and an open stage other than the post-swallowing stage.

It is preferable that the feature value is the number of the frame images in which the stage information is the post-swallowing stage.

It is preferable that the control processor calculates an area of a halation region in the frame image in which the stage information is the initial swallowing-in-progress stage, and the feature value is the area of the halation region.

It is preferable that the control processor calculates a lightness value in the frame image in which the stage information is the post-swallowing stage, and the feature value is the lightness value.

It is preferable that the control processor assigns imaging time point information that is a time point at which the frame image is captured to the frame image, and the feature value is calculated based on a lightness value of each of a plurality of consecutive frame images captured in a specific period among a plurality of time-series frame images included in the swallowing block or a plurality of time-series frame images not included in the swallowing block.

It is preferable that the examination video is obtained by imaging the observation target in a case in which a certain amount of water is swallowed in the video endoscopic examination of swallowing.

It is preferable that the examination video is obtained by imaging the observation target in a case in which a certain amount of swallowing food is swallowed in the video endoscopic examination of swallowing.

It is preferable that the control processor calculates a different feature value depending on a type of the swallowing food to be swallowed.

It is preferable that the control processor receives designation of a start point and an end point for calculating the feature value, and calculates the feature value for a section defined based on the start point and the end point in the examination video.

It is preferable that the control processor performs control of displaying the feature value on a display.

Another aspect of the present invention relates to an operation method of an image processing device, the operation method comprising: a step of acquiring an examination video in which an observation target is imaged in a video endoscopic examination of swallowing; a step of performing recognition processing on the examination video for each frame image, to assign stage information indicating that an image shows which stage of the observation target among a plurality of swallowing stages set in advance to each of the frame images; and a step of calculating a feature value indicating a feature related to swallowing of a subject person having the observation target based on the stage information assigned to each of a plurality of the frame images included in the examination video.

Still another aspect of the present invention relates to a non-transitory computer readable medium for storing a computer-executable program, the computer-executable program causing a computer to implement: a function of acquiring an examination video in which an observation target is imaged in a video endoscopic examination of swallowing; a function of performing recognition processing on the examination video for each frame image, to assign stage information indicating that an image shows which stage of the observation target among a plurality of swallowing stages set in advance to each of the frame images; and a function of calculating a feature value indicating a feature related to swallowing of a subject person having the observation target based on the stage information assigned to a plurality of the frame images included in the examination video.

Still another aspect of the present invention relates to a diagnosis support apparatus comprising: the image processing device described above, in which the diagnosis support apparatus performs a determination related to a swallowing function of the subject person who has undergone the video endoscopic examination of swallowing based on the feature value calculated by the image processing device, and performs control of displaying a result of the determination on a display.

According to the present invention, it is possible to obtain the objective and highly accurate indicator related to the swallowing without taking time and effort.

### BRIEF DESCRIPTION OF THE DRAWINGS

Fig. 1 is a graph showing whether the swallowing is in progress or the swallowing is not in progress based on an observation target shown in a time-series frame image obtained by a video endoscopic examination of swallowing, in which (A) of Fig. 1 is a graph of a subject person A, and (B) of Fig. 1 is a graph of a subject person B.
Fig. 2 is an explanatory diagram showing a configuration of an endoscope system.
Figs. 3A to 3C are explanatory diagrams showing respective stages of the swallowing, in which Fig. 3A shows an oral phase, Fig. 3B shows a pharyngeal phase, and Fig. 3C shows an esophageal phase.
Figs. 4A and 4B are explanatory diagrams showing imaging of a swallowing endoscope, in which Fig. 4A is an explanatory diagram showing a position of a distal end part of the swallowing endoscope, and Fig. 4B is an image diagram of an image captured by the swallowing endoscope.
Fig. 5 is an explanatory diagram showing a swallowing-in-progress stage and a swallowing-not-in-progress stage, which are the stages of the swallowing.
Fig. 6 is an explanatory diagram showing an initial swallowing-in-progress stage and a late swallowing-in-progress stage in the swallowing-in-progress stage.
Fig. 7 is an explanatory diagram showing a swallowing block.
Figs. 8A to 8C are a table showing a feature value related to a swallowing length of each subj ect person and a graph showing the number of frames included in the swallowing block, in which Fig. 8A is a table showing the feature value related to the swallowing length of each subject person, Fig. 8B is a graph related to the number of frames included in the swallowing block of a subject person G, and Fig. 8C is a graph related to the number of frames included in the swallowing block of a subject person C.
Figs. 9A to 9C are a table showing feature values related to whiteout for each subject person and a graph showing the number of whiteout frames included in the swallowing block, in which Fig. 9A is a table showing a feature value related to the number of whiteout frames for each subject person, Fig. 9B is a graph showing the number of whiteout frames included in the swallowing block of the subject person B, and Fig. 9C is a graph showing the number of whiteout frames included in the swallowing block of the subject person C.
Fig. 10 is an explanatory diagram showing a frame image captured in a specific period in the swallowing block.
Fig. 11 is an explanatory diagram showing a frame image captured in a specific period that is not included in the swallowing block.
Fig. 12 is an explanatory diagram showing a configuration of a diagnosis support apparatus.
Fig. 13 is a flowchart showing a flow of the video endoscopic examination of swallowing.
Fig. 14 is a flowchart showing a flow of processing performed by the image processing device and the diagnosis support apparatus.

### DESCRIPTION OF THE PREFERRED EMBODIMENTS

An example of an embodiment of an image processing device and the like according to the embodiment of the present invention will be described. First, a process leading to obtaining the embodiment described below will be described. In a video endoscopic examination of swallowing, a doctor observes an image of a swallowing movement obtained through an endoscope and evaluates the movement. However, the diagnosis is mainly a subjective evaluation of the doctor, and there has been no method of determining, evaluating, or the like a swallowing endoscopic image with an objective numerical value or indicator.

For example, a system is disclosed, which determines whether the swallowing is in progress or the swallowing is not in progress from the examination image (JP2022-179218A or JP2022-179222A). JP2022-179218A describes that whether the swallowing is in progress or the swallowing is not in progress is automatically determined from the examination image. In addition, JP2022-179222A describes that whether the swallowing is in progress or the swallowing is not in progress in the video of the swallowing endoscope is determined for each frame. However, the generation of diagnosis support information using the determination result is not disclosed.

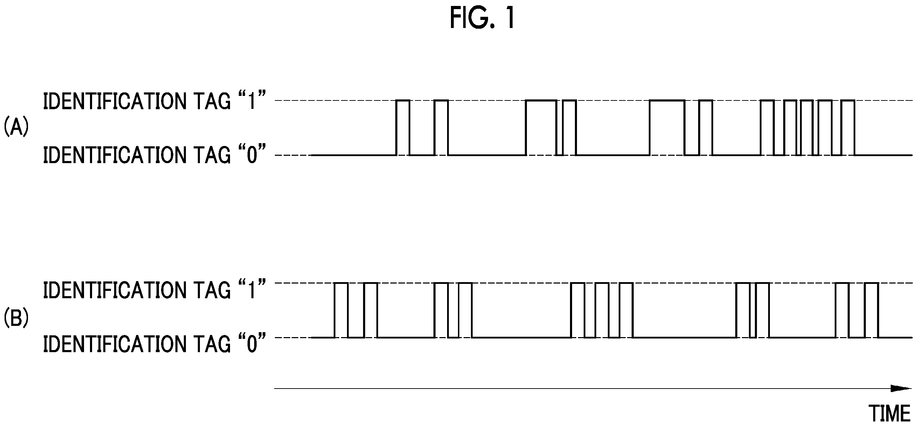

In the course of studying the generation of the diagnosis support information using the determination result of whether the swallowing is in progress or the swallowing is not in progress in an examination video obtained by a swallowing endoscope for each frame, in a case in which a graph is drawn, for example, as shown in Fig. 1, with a time point of imaging for each frame image in the time-series frame images on a horizontal axis and whether the swallowing is in progress (= 0, identification tag "0") or the swallowing is not in progress (= 1, identification tag " 1") as a determination result determined by the doctor based on the frame image on a vertical axis, (A) of Fig. 1, which is a graph of a subject person A, and (B) of Fig. 1, which is a graph of a subject person B, are obtained, and it is found that different features are observed depending on a swallowing state of a subject person. Therefore, as a result of more detailed examination of the determination of whether the swallowing is in progress or the swallowing is not in progress, it is found that different features may be observed depending on the swallowing state of the subject person.

Based on the above-described studies, the present invention provides an image processing device, an operation method of an image processing device, an image processing program, and a diagnosis support apparatus capable of obtaining an objective and highly accurate indicator related to the swallowing without taking time and effort.

The image processing device according to the embodiment of the present invention comprises a control processor. The control processor acquires an examination video in which an observation target is imaged in a video endoscopic examination of swallowing, performs recognition processing on the examination video for each frame image, to assign one stage information indicating that an image shows which stage among a plurality of swallowing stages set in advance to each of the frame images, and calculates a feature value indicating a feature related to swallowing of a subject person who has undergone the video endoscopic examination of swallowing based on the stage information assigned to each of a plurality of the frame images included in the examination video.

The video endoscopic examination of swallowing is an examination performed for the purpose of evaluation of functional abnormalities in a pharyngeal phase of the swallowing movement, evaluation of organic abnormalities, confirmation of the effects of compensatory methods and rehabilitation techniques, education and guidance for patients, families, and medical staff, and the like (Japanese Journal of Swallowing Rehabilitation, Vol. 17, pp. 87 to 99, 2013, Japanese Society of Swallowing Rehabilitation, Medical Committee: Procedure for video endoscopic examination of swallowing 2012 revised (revised version)), and is an examination for performing diagnosis and the like related to the swallowing of the subject person by observing endoscopic images obtained by imaging a pharyngeal part, a laryngeal part, and the like of the subject person in the swallowing movement by inserting a relatively thin endoscope from any one of a left nasal cavity or a right nasal cavity of the subject person.

Specifically, the image processing device is a processing computer comprising a control processor. The image processing device may be incorporated in a processor device included in an endoscope system used for the video endoscopic examination of swallowing, or may be a device different from the processor device. The image processing device may be disposed at a remote position of the endoscope system by connecting the endoscope system and the image processing device via a network in a communicable manner.

The image processing device acquires an examination video in which an observation target is imaged in a swallowing endoscope. That is, the image of the video endoscopic examination of swallowing obtained by the endoscope system is input to the processing computer. The image is a set of frame images consecutively acquired in time series, and is a so-called video.

In the examination video which is the video acquired by the image processing device, still image data may be input in order, or video data may be collectively input and then stored in a recording device in the processing computer, and then recognition processing or the like may be performed. The still image data can be handled in the same manner as the frame image constituting the video data. Therefore, the frame image includes an image for each frame constituting the examination video, and the still image data.

In the examination video, the observation target is imaged at a predetermined frame rate. The frame rate is the number of still images acquired by an imaging element provided in the endoscope system in one second. The frame rate in the swallowing endoscope can be set in advance, and for example, 30 frames/second or 60 frames/second is common, and there are some cases in which a setting of 120 frames/second can be made by using a faster image sensor. The image for each frame in the examination video captured at such a frame rate is a frame image. It should be noted that, in a more high-performance endoscope system, a higher frame rate may be realized, and in some cases, the observation target may be imaged at a lower frame rate.

The examination video includes a plurality of time-series frame images. By referring to the frame rate set in advance, an imaging time point at which a specific frame image is captured, an imaging period during which a plurality of specific frame images are captured, or the like can be calculated. For example, the imaging period in which the frame image is captured can be calculated by the number of consecutive frame images, and the imaging time point at which the frame image is captured can be obtained based on an order in which the specific frame image is captured, by using the imaging time point of the first frame image in the examination as the start time point of the examination.

The processing computer comprises a classifier that is a learning model that has been trained through machine learning using deep learning in advance. The classifier performs recognition processing for each frame image on the examination video. By the recognition processing via the classifier, stage information indicating that an image shows which stage among a plurality of swallowing stages set in advance is assigned to each of the frame images. In usual, one stage information is assigned to each frame image.

The classifier is trained to receive the input of the examination video and classify or determine which stage of the observation target among the swallowing stages is imaged in each frame image. The stage information indicating the swallowing stage is a predetermined number of stages. Therefore, the classifier is a classifier that outputs classification of which swallowing stage of the predetermined number of swallowing stages the frame image is an image in which the observation target of the swallowing stage is captured in a case in which the frame image in which the stage information is unknown is input.

The classifier may be any classifier using any architecture as long as the classifier can determine which stage of the observation target among the swallowing stages is imaged, for each frame image. In the classifier for achieving the purpose of classifying the image in accordance with what is shown, a convolutional neural network (CNN) trained by training data consisting of a frame image in which the observation target of each swallowing stage is shown and a correct answer of each swallowing stage shown in the frame image can be preferably used.

The CNN is a type of the deep learning, and can extract local patterns and features of an image and hierarchically learn the extracted local patterns and features, so that the CNN can exert high performance in an image classification task of inputting a frame image in which the swallowing stage is unknown and outputting which swallowing stage of the observation target is imaged. Examples of the content of the high performance in the image classification task of the CNN include accuracy, which provides an accurate classification result, generalization, which can exert good performance even for similar data or new data not included in the training data, robustness, which exerts robust performance against various conversions or noise on the image, feature learning, which learns the local features and hierarchical structure of the image, and parameter efficiency, which can effectively reduce the number of parameters of the model.

By the recognition processing via the classifier, a recognition result is obtained, which indicates that the image shows which stage of the observation target shown in the frame image among the plurality of swallowing stages set in advance. Then, the stage information indicating the swallowing stage, which is the recognition result, is assigned to each of the frame images.

The classification to be learned by the classifier includes at least "during a period in which the swallowing is in progress" and "during a period in which the swallowing is not in progress". Therefore, the plurality of swallowing stages set in advance include at least "during a period in which the swallowing is in progress" and "during a period in which the swallowing is not in progress".

The image processing device calculates a feature value indicating a feature related to the swallowing of the subject person who has undergone the video endoscopic examination of swallowing, based on the stage information assigned to each of the plurality of frame images included in the examination video of the one video endoscopic examination of swallowing. In a case in which the stage information to be assigned to the frame image is "during a period in which the swallowing is in progress" and "during a period in which the swallowing is not in progress", the feature value can be the number of frame images in which the stage information is "during a period in which the swallowing is in progress" in the examination video. For example, by performing comparison with the subject person having a normal swallowing function by aligning the number of times of swallowing indicated during the examination, it is possible to objectively understand that the swallowing is taking a long time in a case in which there is a larger number of frame images in which the stage information is "during a period in which the swallowing is in progress", whereas it is possible to objectively understand that the swallowing is not taking a long time in a case in which there is an equal or smaller number of frame images in which the stage information is "during a period in which the swallowing is in progress".

The feature value is displayed on a display or the like of the endoscope system. In addition, since the stage information such as "during a period in which the swallowing is in progress" is assigned to each of the frames constituting the examination video, the doctor can perform a more precise diagnosis while displaying the feature value that is an indicator of the diagnosis on the display along with the image obtained by imaging the observation target at the specific stage.

In addition to the display of the feature value, the display may display the examination video with the superimposed stage information, or may display, in time series, each of the specific frames included in the examination video and superimposed with the stage information in accordance with an instruction issued by the user such as the doctor. Each specific frame may be extracted from the examination video image, and for example, a frame having specific stage information, a frame captured from a frame captured at a specific time point to a designated time point, or the like may be extracted and displayed.

With the above-described configuration, the image processing device can classify a swallowing state with an objective indicator by using the feature value obtained by quantifying the feature related to the swallowing of the subject person. Specifically, the feature value is calculated by inputting the examination video to the image processing device, and the feature value is displayed on the display. The stage information from which the feature value is calculated is the frame image subjected to the recognition processing through the machine learning. Therefore, with the image processing device, it is possible to obtain an objective and highly accurate indicator related to the swallowing without taking time and effort.

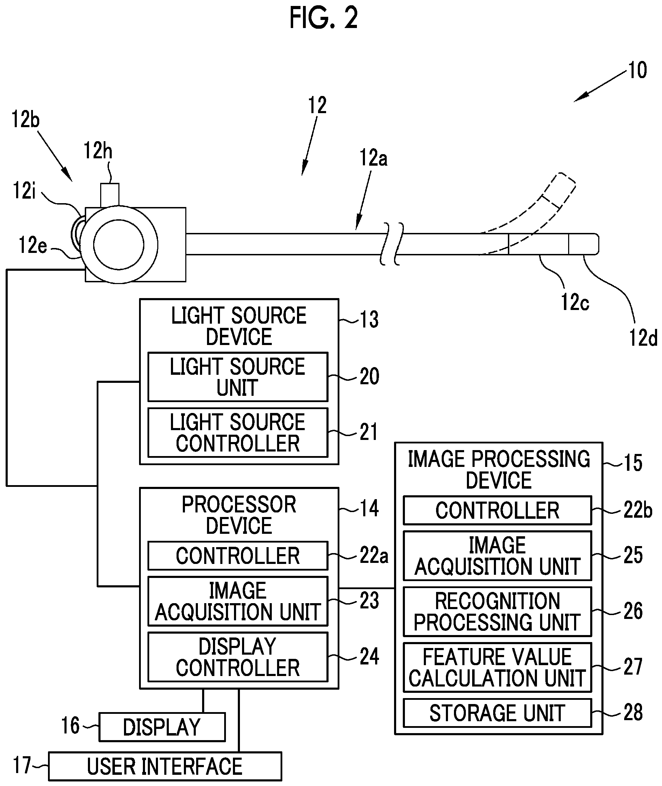

Hereinafter, the embodiment will be further described based on the drawings. As shown in Fig. 2, the endoscope system 10 comprises an endoscope 12, a light source device 13, a processor device 14, an image processing device 15, a display 16, and a user interface 17. The endoscope 12 is an endoscope of a so-called electronic endoscope used for the video endoscopic examination of swallowing. The endoscope 12 is optically connected to the light source device 13, and is electrically connected to the processor device 14.

The endoscope 12 includes an insertion part 12a to be inserted into a body of an observation target, an operating part 12b provided at a base end portion of the insertion part 12a, and a bendable part 12c and a distal end part 12d provided on a distal end side of the insertion part 12a. The bendable part 12c is operated in a bendable manner by operating an angle knob 12e of the operating part 12b. As the bendable part 12c is operated in a bendable manner, the distal end part 12d is made to face in a desired direction. In the video endoscopic examination of swallowing, the insertion part 12a is inserted from any one of the left nasal cavity or the right nasal cavity of the subject person.

An imaging optical system for forming a subject image and an illumination optical system for irradiating a subject with illumination light are provided in the endoscope 12. The subject as the observation target is an in-vivo structure related to the swallowing movement, and specifically, is a pharyngeal part and a laryngeal part. The illumination light passes through the insertion part 12a of the endoscope 12 via a light guide and is emitted toward the subject from the distal end part 12d via an illumination lens of the illumination optical system. It should be noted that, in a case in which the light source unit 20 is built in the distal end part 12d of the endoscope, the light source unit 20 emits light to the subject via the illumination lens of the illumination optical system without passing through the light guide.

The imaging optical system is provided with an objective lens and an imaging sensor. The light from the observation target due to the irradiation with the illumination light is incident on the imaging sensor through the objective lens and a zoom lens. Therefore, an image of the observation target is formed on the imaging sensor. The zoom lens is a lens for magnifying the observation target, and is moved between a telephoto end and a wide end by operating a zoom operation unit 12i. The imaging sensor may be disposed in the distal end part 12d of the endoscope 12, or may be a so-called fiberscope that uses a fiber bundle in the insertion part of the endoscope 12 and located at the end of the insertion part 12a on the operating part side.

The imaging sensor is a complementary metal-oxide-semiconductor (CMOS) sensor, a CCD sensor, or the like. An examination image is generated based on an image signal detected by the imaging sensor. The imaging sensor may include a monochrome imaging sensor in which a color filter that converts sensed light into a monochrome image signal is not provided, in addition to a color imaging sensor in which a color filter (Bayer filter or the like) that converts the sensed light into a color image signal is provided. It should be noted that the color imaging sensor may convert the sensed light into a CMY signal, instead of an RBG signal.

In a case in which a color image is acquired, the image signal includes a B image signal output from a B pixel of the imaging sensor, a G image signal output from a G pixel, and an R image signal output from an R pixel. The image signal is input to the image acquisition unit 23 of the processor device 14 and is acquired as an examination image that is a monochrome image or the color image. The examination image acquired by the image acquisition unit 23 is output to the image acquisition unit 25 of the image processing device 15. The examination image output to the image acquisition unit 25 is output to a recognition processing unit 26. The examination image is a still image captured during the video endoscopic examination of swallowing or the examination video captured during the video endoscopic examination of swallowing.

The operating part 12b is provided with a still image acquisition instruction switch 12h that is used to issue an instruction to acquire the still image of the observation target and a zoom operation unit 12i that is used to operate the zoom lens, in addition to the angle knob 12e.

The light source device 13 generates the illumination light. The processor device 14 performs system control of the endoscope system 10, image processing for displaying the image on the display 16 on the image signal output from the endoscope 12, and the like. The display 16 is a display unit that displays an image captured by the endoscope 12. The user interface 17 is an input device used to input settings and the like to the processor device 14 and the like. It should be noted that the display 16 and the user interface 17 also perform an output of an image or the like from the image processing device 15, an input of a setting input, or the like. In a case in which the display 16 comprises a touch panel, the touch panel is included in the user interface 17.

The light source device 13 comprises a light source unit 20 that emits the illumination light, and a light source controller 21 that controls an operation of the light source unit 20. The light source unit 20 emits the illumination light for illuminating the subject. The light source unit 20 includes a light source, such as a laser diode, a light emitting diode (LED), a xenon lamp, or a halogen lamp. The light source may comprise a plurality of light sources having different wavelengths. The light source controller 21 controls turning-on or turning-off of each light source constituting the light source unit 20, an amount of light emitted from each light source, and the like. As a result, the illumination light having a specific amount of light or a specific wavelength can be emitted. In usual, white illumination light that allows the observation target to be observed in a natural tint is emitted.

It should be noted that the light source unit 20 may be built in the endoscope 12. In addition, the light source controller 21 may be built in the endoscope 12 or may be built in the processor device 14. The white illumination light includes so-called pseudo-white color which is obtained by mixing violet light V, blue light B, green light G, or red light R and which is substantially equivalent to white color in the imaging of the subject using the endoscope 12. A light source that emits ultraviolet light or infrared light for special light observation may be further included. Further, the light source unit 20 includes, as necessary, an optical filter or the like that adjusts a wavelength range, a spectrum, a light amount, or the like of the illumination light.

The light source unit 20 may perform irradiation by sequentially switching the wavelength to be emitted at a high speed, for example, the blue light B, the green light G, and the red light R, the image sensor may acquire an image of each illumination light color by the monochrome sensor or the color sensor, and the processor may combine the images to generate a white image. In the light source unit 20, as a mechanism for switching the wavelength to be emitted at a high speed, there is a method of mechanically switching a plurality of color filters of different colors with respect to a white light source such as a xenon lamp, or a method of electronically switching ON/OFF of a plurality of light emitting diodes (LEDs) that emit different colors.

The processor device 14 includes a controller 22a, an image acquisition unit 23, and a display controller 24. The processor device 14 is a computer, and comprises a central processing unit (CPU), a memory, and the like. The CPU provided in the computer is an example of a processor. In the processor device 14, a program in a program memory is operated by the controller 22a composed of a processor, to implement the functions of the controller 22a, the image acquisition unit 23, the display controller 24, and the like.

The image processing device 15 includes a controller 22b, the image acquisition unit 25, the recognition processing unit 26, a feature value calculation unit 27, a storage unit 28, and the display controller 24. The image processing device 15 is a computer, and comprises a CPU, a memory, and the like. The CPU provided in the computer is an example of a control processor. In the image processing device 15, a program in the memory included in the computer is operated by the controller 22b composed of the control processor, to implement the functions of the controller 22b, the image acquisition unit 25, the recognition processing unit 26, the feature value calculation unit 27, and the storage unit 28. It should be noted that the image processing device 15 and/or the light source controller 21 may be included in the processor device 14.

As described above, the image acquisition unit 25 acquires the examination video in which the observation target is imaged in the video endoscopic examination of swallowing using the endoscope system 10. The examination video may be acquired in real time, or the examination video acquired in the examination performed in advance may be stored in the storage unit 28, and then the examination video may be acquired from the storage unit 28 below.

The recognition processing unit 26 performs the recognition processing of assigning the stage information to the examination video for each frame image. In the recognition processing, it is recognized that the image shows which stage of the observation target among the plurality of swallowing stages set in advance, based on the image shown in each of the frame image, and the stage information which is the recognition result is assigned to each of the frame images.

A known technique can be used as a method of the recognition processing, and it is preferable to use the classifier as described above. For example, a classifier using a technique, such as semantic segmentation, may be used. In addition, the architecture may not be limited to using supervised learning, and an architecture using unsupervised learning may be used as long as the stage information can be appropriately assigned to the frame image by the image recognition processing, and for example, clustering, generative adversarial networks (GANs), or the like may be used. As described above, in order to recognize the frame image and to assign appropriate stage information, a technique used in the machine learning, such as the selection of the architecture of the learning model or the learning method, whether or not to use a plurality of CNNs, or whether or not to perform branching, may be appropriately used.

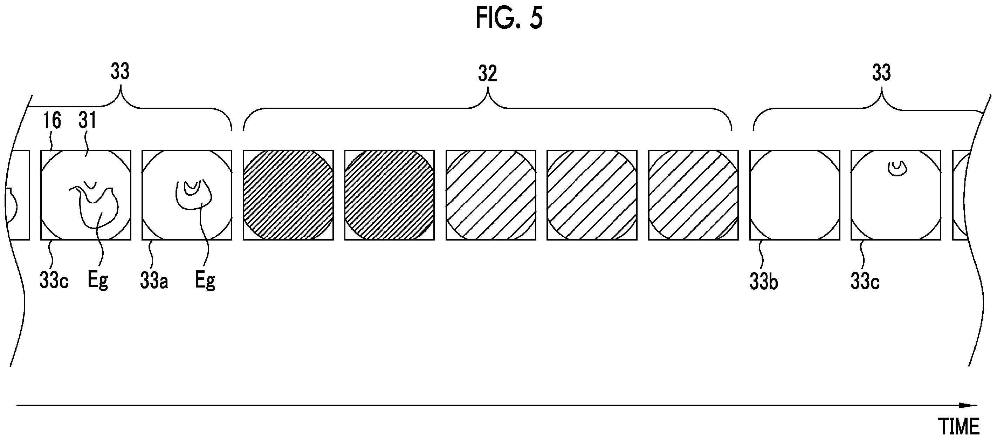

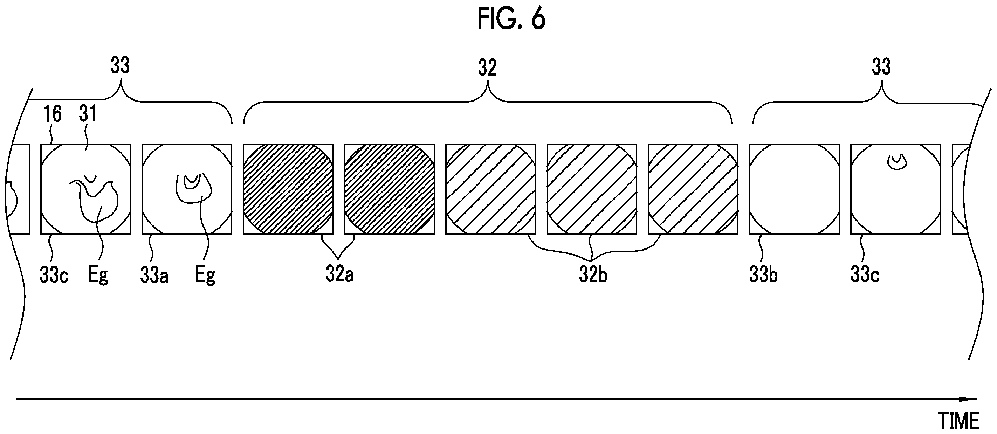

As one of the techniques used in the machine learning, a cascade type classifier may be used, which is a method of improving the efficiency and speed of detection by applying a plurality of classifiers stepwise. In the deep learning, stepwise learning, recognition processing, or the like may be performed, for example, learning of detecting an initial swallowing-in-progress stage (see Fig. 6, whiteout) in a frame image is performed first, learning of detecting a late swallowing-in-progress stage is performed next, learning of detecting a frame image in a case of swallowing water, a swallowing food, and other types of specific swallowing food is performed, and learning of detecting a frame image of a pre-swallowing stage and a post-swallowing stage (see Fig. 5, blackout) is performed. By performing the recognition processing on the frame image via the cascade type classifier, effects such as high-speed detection, high detection accuracy, and resource saving can be obtained.

Time-series filtering may be applied as one of the techniques used in the machine learning. The time-series filtering is a process of extracting or converting a specific pattern or trend of time-series data by using the series of methods or algorithms in the machine learning, and is used for noise removal, trend analysis, detection of abnormal values, and the like of the time-series data. Since the frame image is the time-series data, the time-series filtering can be suitably used.

It should be noted that such techniques used in the machine learning may be used in combination. In a case in which the cascade type classifier and the time-series filtering are used in combination, the case is preferable since the recognition processing can be performed at high speed and with high accuracy.

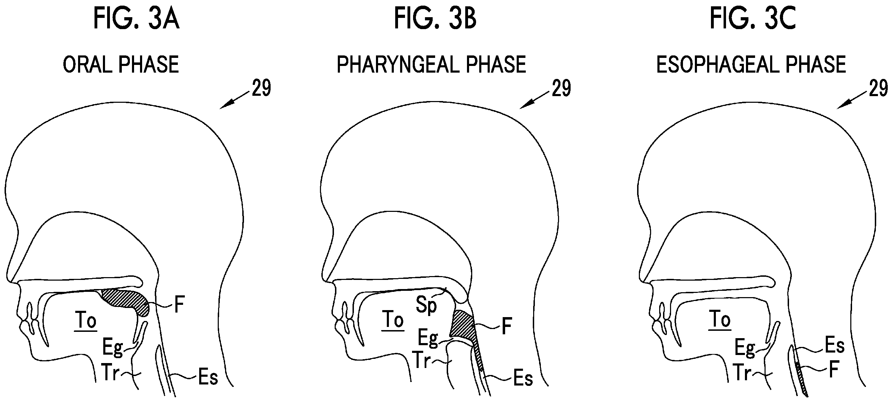

Here, the swallowing stage will be described. The swallowing refers to the series of motions of putting food or drink into the mouth, chewing the food or drink, swallowing the food or drink, and sending the food or drink to the esophagus. As shown in Fig. 3, in a case in which a head 29 of a person facing forward is viewed from the left side of the person and the oral cavity, the pharynx, and the like are represented, the swallowing movement is divided into an "oral phase" (Fig. 3A) in which food F is carried from the oral cavity to the pharynx mainly by the movement of a tongue To, a "pharyngeal phase" (Fig. 3B) in which the food F is carried from the pharynx to an esophagus Es by a swallowing reflex, and an "esophageal phase" (Fig. 3C) in which the food F is carried from the esophagus Es to the stomach by peristaltic movement of the esophagus. During the swallowing, in order to direct the food F toward the esophagus Es and to prevent the food F from flowing into the trachea Tr, the epiglottis Eg that plays a role of covering the trachea Tr closes the entrance (glottis) of the trachea Tr by a reflex movement. A soft palate Sp, which is a ceiling of the oral cavity, also moves backward to close a passage between the oral cavity and the nasal cavity, thereby preventing the food F from entering the nasal cavity.

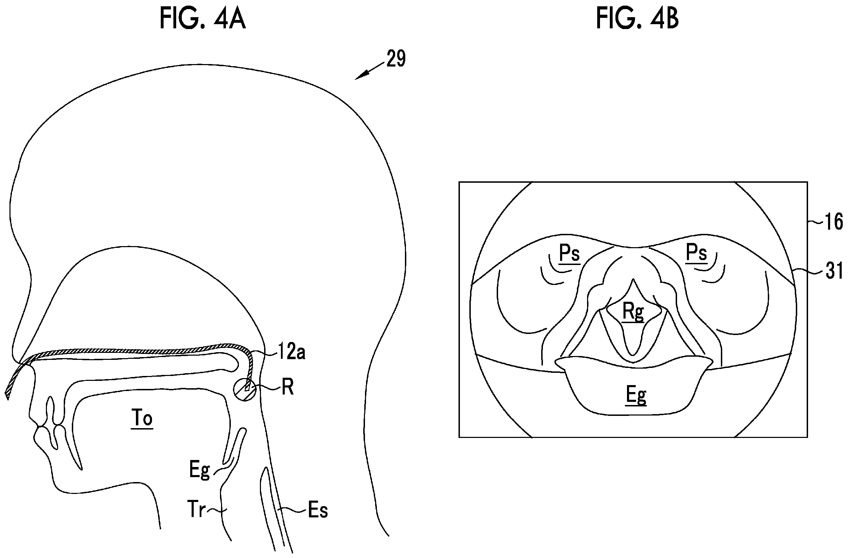

The examination video acquired in the video endoscopic examination of swallowing is acquired by inserting the insertion part 12a of the endoscope 12 from the nasal cavity to the pharynx and imaging the examination image such that the distal end part 12d of the endoscope is located near a position R of an oropharyngeal part as shown in Fig. 4A. It is preferable that the frame image included in the examination video in this case includes anatomical structures such as the epiglottis Eg, a rima glottidis Rg, and right and left pyriform sinuses Ps as shown in a frame image 31 in Fig. 4B. The rima glottidis Rg is a space between the left and right folds constituting the vocal cords. Hereinafter, although a case will be described in which the distal end of the endoscope is disposed in the oropharyngeal part, as long as the swallowing stage can be understood, the distal end may be disposed in other parts, such as the nasopharyngeal part, the nasopharyngeal part, the hypopharyngeal part, or the laryngeal part, to perform the swallowing determination.

In the examination image captured in the video endoscopic examination of swallowing, the subject having a shape, a part, or the like is captured in accordance with the swallowing stage, and thus the examination image is an image having features on different images. The plurality of swallowing stages or the stage information set in advance based on the examination image need only indicate the stage of the swallowing movement, and may be set as desired in accordance with the purpose of acquiring any feature value or the like.

The stage information is information indicating the series of swallowing movements in stages. In the swallowing movement, the swallowing is started from a swallowing-not-in-progress stage, the swallowing ends through a swallowing-in-progress stage, and the transition is made again to the swallowing-not-in-progress stage. The series of swallowing movements from the start of the swallowing to the end of the swallowing is defined as one swallowing. In a case in which the swallowing movement occurs again from the swallowing-not-in-progress stage, the series of swallowing movements in which the swallowing is started from the swallowing-not-in-progress stage is repeated again. The examination video includes the frame images in a case in which the swallowing movement is performed a plurality of times.

As shown in Fig. 5, in a case in which the examination images in which the swallowing movement is imaged at a standard frame rate of 30 frames/second are arranged in time series, the examination images are images having different features based on the swallowing stage. In a swallowing-not-in-progress stage 33, which is a stage during a swallowing-not-in-progress period in which the swallowing is not in progress, the examination image shown on the display 16 includes the frame image 31, and is an image in which the epiglottis Eg is shown. It should be noted that, in the drawings, in a case in which there are a plurality of the same components, reference numerals may not be added to all of the components in order to avoid complication of the drawings.

In the swallowing-in-progress stage 32, which is a stage during a period in which the swallowing is in progress, first, the pharyngeal contraction during a period in which the swallowing is in progress is started, and movements such as lifting of the epiglottis and contraction of the pharyngeal mucous membrane are observed in a short time. Thereafter, in a case in which the pharyngeal contraction is strengthened, the mucous membrane comes into contact with the distal end part 12d, and a visual field of the endoscope is not visible. Therefore, in the frame image 31, a wide part is covered with white halation or a slightly dark tone. A white halation region of the frame image 31 is also referred to as whiteout (see Fig. 6, the initial swallowing-in-progress stage). Thereafter, the entire screen is blurred and covered with a slightly dark color (see Fig. 6, the late swallowing-in-progress stage). In the swallowing-not-in-progress stage 33, in the image immediately after the end of the swallowing, the mucous membrane that has been close to the distal end part 12d disappears, and an image in which the entire screen is dark is obtained by automatic exposure adjustment of the endoscope (see Fig. 5, a post-swallowing stage 33b). Then, the epiglottis Eg is shown again.

It is preferable that the swallowing stage distinguished from the examination image is set to the swallowing-in-progress stage 32 and the swallowing-not-in-progress stage 33. Therefore, the stage information can be the swallowing-in-progress stage 32 indicating that the observation target is swallowing and the swallowing-not-in-progress stage 33 indicating that the observation target is not swallowing.

It is preferable that the swallowing-not-in-progress stage 33 includes a pre-swallowing stage 33a that is the swallowing-not-in-progress stage 33 immediately before the swallowing-in-progress stage 32, the post-swallowing stage 33b that is the swallowing-not-in-progress stage 33 immediately after the swallowing-in-progress stage 32, and an open stage 33c that is the swallowing-not-in-progress stage 33 excluding the pre-swallowing stage 33a and the post-swallowing stage 33b.

In the stage information, it is preferable that the swallowing-in-progress stage 32 indicating that the observation target is swallowing includes an initial swallowing-in-progress stage indicating that the observation target is in an initial stage of the swallowing and a late swallowing-in-progress stage indicating that the observation target is in a late stage of the swallowing.

As shown in Fig. 6, in the initial swallowing-in-progress stage 32a, an image called whiteout, which is seen in about 2 frames immediately after the pre-swallowing stage 33a in which the movement of the pharyngeal part and the associated blur are seen, is obtained. Thereafter, an image in which the entire screen is covered with a blurred and slightly dark tone continues for several frames, and a late swallowing-in-progress stage 32b is assigned as the stage information to these images.

As described above, as the stage information of the series of swallowing movements, the stage information of the open stage 33c, the pre-swallowing stage 33a, the initial swallowing-in-progress stage 32a, the late swallowing-in-progress stage 32b, the post-swallowing stage 33b, and the open stage 33c can be set in time series. The number of frames in each stage varies depending on the individual swallowing movement, and some of the stage information may not be seen in some cases. The number of frames in each stage, the stage information that is not seen, and the like can also be used as one of the feature values for understanding the swallowing state.

The feature value calculation unit 27 calculates the feature value indicating the feature related to the swallowing of the subject who has undergone the video endoscopic examination of swallowing, based on the stage information assigned to each of the frame images 31. The display controller 24 performs control of displaying the feature value on the display 16.

It is preferable that the feature value is the number of frame images 31 which are included in the examination video of one video endoscopic examination of swallowing and in which the stage information is the post-swallowing stage 33b. In the post-swallowing stage 33b, since the mucous membrane that has been present near the distal end part 12d disappears, an image in which nothing is shown on the screen and the entire screen is dark is often obtained. The image in the post-swallowing stage 33b can be blacked out and used as an image in a stage in which the swallowing movement ends. Therefore, the number of frame images 31 in which the stage information is the post-swallowing stage 33b can be used as a feature value representing the number of swallowing movements.

It should be noted that the number of frame images 31 in which the stage information is the post-swallowing stage 33b may be the number of blackout frames extracted for the frame images 31 in a period from the start to the end of the examination, or may be the number of blackout frames extracted in a specific period. The specific period can be optionally set.

The feature value may be a lightness value in the frame image 31 in which the stage information is the post-swallowing stage 33b. In this case, the feature value calculation unit 27 calculates the lightness value for the frame image 31 to which the stage information of the blackout of the post-swallowing stage 33b is assigned. As the lightness value, the B image signal, the G image signal, or the R image signal of the color image of the frame image 31 may be used, a brightness value may be used, or both of the B image signal, the G image signal, or the R image signal and the brightness value may be used. Statistical information such as an average value using the lightness value of the frame image 31 having the stage information of a plurality of post-swallowing stages 33b may be used as the feature value, or the lightness value of the frame image 31 having the stage information of a specific post-swallowing stage 33b may be used as the feature value.

By using the lightness value of the frame image 31 in the post-swallowing stage 33b as the feature value, it is possible to obtain an indicator such as a speed at which the pharynx that has contracted at the end of the swallowing is opened, a contraction pressure of the pharyngeal contraction, or a swallowing pressure. The lightness value tends to be relatively low in a case in which the pharynx is quickly opened, and the lightness value tends to be high in a case in which the opening of the pharynx is slow. In addition, the lightness value tends to be high in a case in which the contraction pressure, the swallowing pressure, and the like of the pharynx are weak, and the lightness value tends to be low in a case in which the contraction pressure, the swallowing pressure, and the like are strong.

It is preferable that the feature value is the number of the frame images 31 in which the stage information is the initial swallowing-in-progress stage 32a. The initial swallowing-in-progress stage 32a is a whiteout image, and this image can be used as an image indicating a stage of starting the swallowing movement. Therefore, the number of frame images 31 in which the stage information is the initial swallowing-in-progress stage 32a can be used as a feature value representing the number of swallowing movements.

The feature value may be an area of the halation region in the frame image 31 in which the stage information is the initial swallowing-in-progress stage 32a. In this case, the feature value calculation unit 27 extracts the frame image 31 in which the stage information is the initial swallowing-in-progress stage 32a, and calculates the area of the halation region in the frame image 31. For the calculation of the area of the halation region, the B image signal, the G image signal, or the R image signal of the color image of the frame image 31 may be used, the brightness value may be used, or both of the B image signal, the G image signal, or the R image signal and the brightness value may be used. The whiteout frame image 31, which is the frame image 31 in the initial swallowing-in-progress stage 32a, can be set to have a pixel value equal to or more than a threshold value set in advance in the lightness value or the brightness value for each pixel.

By using the area of the halation region of the frame image 31 in the initial swallowing-in-progress stage 32a as the feature value, it is possible to use the area of the halation region as an indicator such as the contraction speed of the pharynx at the start of the swallowing, the contraction pressure of the pharyngeal contraction, and the contraction shape of the pharynx. The halation region tends to be relatively high in a case in which the pharynx contracts strongly or quickly, and the halation region tends to be relatively low in a case in which the pharyngeal contraction is weak or slow.

It should be noted that, similar to the blackout image, in the whiteout image, the number of frame images 31 in which the stage information is the post-swallowing stage 33b or the area of the halation region may be the number of whiteout frame images 31 extracted for the frame images 31 in a period from the start to the end of the examination, or may be the number of whiteout frame images 31 extracted in a specific period. The specific period can be optionally set.

In calculating the feature value, it is preferable that a specific period as a target of the feature value calculation is set as one swallowing motion. The feature value calculation unit 27 detects a swallowing block 34 that is a group of the frame images in which one swallowing motion of the observation target is imaged. It is preferable that the swallowing block 34 includes a plurality of consecutive frame images 31 in which the stage information is the swallowing-in-progress stage 32 (see Fig. 6).

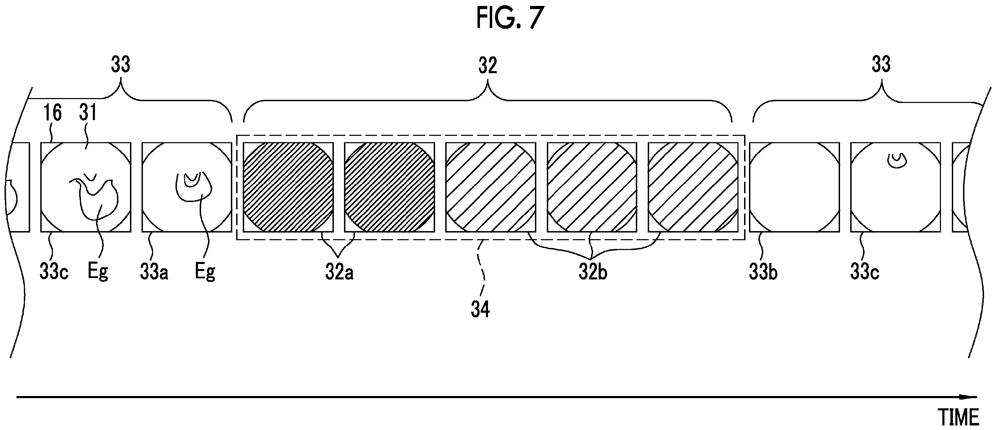

As shown in Fig. 7, in a case in which the frame images 31 included in the examination video are arranged in time series, one swallowing block 34 consists of a plurality of consecutive frame images 31 in the swallowing-in-progress stage 32 from the frame image 31 immediately after the pre-swallowing stage 33a to the frame image 31 immediately before the post-swallowing stage 33b. In addition, one swallowing block 34 consists of a plurality of frame images 31 in which the initial swallowing-in-progress stage 32a and the late swallowing-in-progress stage 32b are consecutive in the frame image 31 of the swallowing-in-progress stage 32. The swallowing block 34 can be detected by any of these detection methods. Therefore, by using a combination of the plurality of pieces of stage information, it is possible to appropriately set and count one swallowing motion in accordance with various swallowing states such as a state in which the frame image 31 in the post-swallowing stage 33b is not visible.

It is preferable to set the feature value related to the appearance of the swallowing block 34 itself. Specifically, it is preferable that the feature value is the number of swallowing blocks 34. The number of swallowing blocks 34 can be the same as the number of times of swallowing. The number of swallowing blocks 34 may be the number extracted by targeting the frame images 31 in a period from the start to the end of the examination, or may be the number extracted by targeting the frame images 31 in a specific period. The specific period can be optionally set. The number of times of swallowing in the specific period can be used as one of useful information for diagnosing the swallowing state.

It is preferable that the feature value is a basic statistic based on the number of frame images 31 included in each of the swallowing blocks 34. In a case in which the frame rate does not vary at a predetermined rate, the number of frame images 31 included in each of the swallowing blocks 34 can be set as a temporal length of one swallowing motion. Since the swallowing motion is performed a plurality of times in one video endoscopic examination of swallowing, a plurality of frame images 31 are obtained in each swallowing movement, and the basic statistic is calculated for these numbers to obtain the feature of swallowing.

As the basic statistic, an average value (mean), which is an indicator indicating a central tendency of data, a median value, which is an indicator indicating a central tendency of data that is less likely to be affected by outliers, a mode value, which is an indicator indicating a central tendency of discrete data, a variance, which is an indicator indicating a degree of dispersion of data, a standard deviation, which is used to evaluate a variation or reliability of data in the same manner as the variance, a range, which is an indicator indicating a difference between a maximum value and a minimum value of a data set, a maximum value, a minimum value, or the like can be optionally used in accordance with the purpose of indicating the swallowing feature.

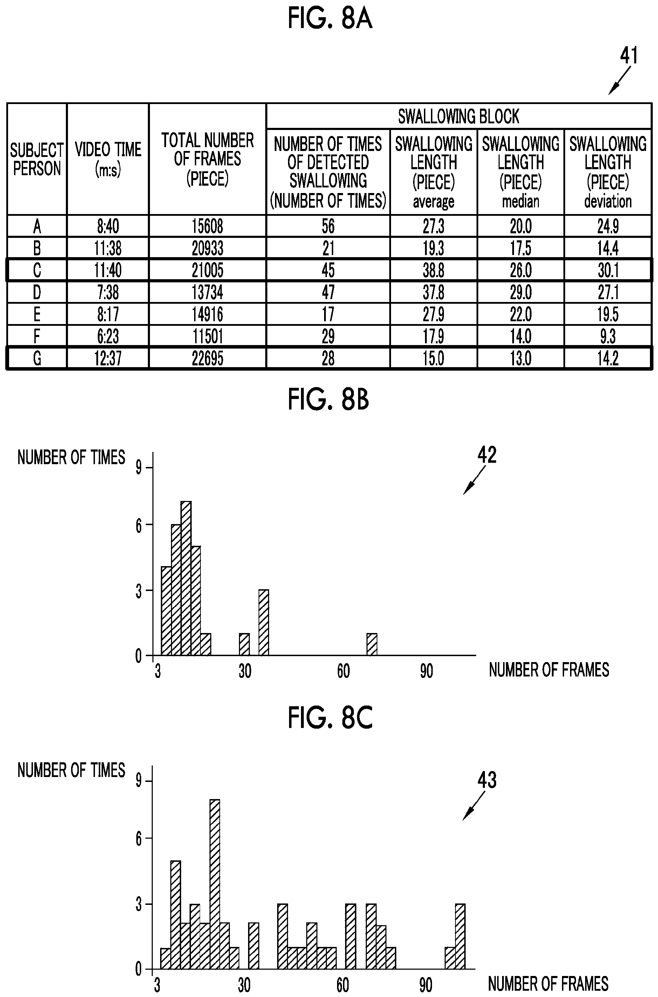

As shown in Fig. 8A, in the table, a video time (unit: min:sec) from the start of the examination to the end of the examination and the total number of frames (unit: piece) are summarized for the subject persons A to G who have undergone the video endoscopic examination of swallowing, and the number of times of detected swallowing (unit: number of times) and the swallowing length/average (unit: piece) as the average of the number of frames included in each swallowing block 34, the swallowing length/median (unit: piece) as the median value of the number of frames included in each swallowing block 34, and the swallowing length/deviation (unit: piece) as the variance of the number of frames included in each swallowing block 34 as the basic statistics for the number of frames included in the each swallowing block 34 are summarized for the swallowing block 34 detected within the video time.

As shown in Table 41, in the swallowing block 34, in a case of focusing on the swallowing length/average, it is found that there is a difference of 2 times or more in the numerical value depending on the subject person. Therefore, as shown in Fig. 8B, in a case in which a histogram 42 indicates how many times swallowing has occurred at a predetermined swallowing length (the number of frames included in the swallowing block 34) for each swallowing block 34 of the subject person G having the shortest swallowing length/average, it is clarified that a large number of times of swallowing having a relatively short swallowing length are performed.

On the other hand, as shown in Fig. 8C, in a case in which a histogram 43 indicates how many times swallowing has occurred at a predetermined swallowing length (the number of frames included in the swallowing block 34) for each swallowing block 34 of the subject person C having the largest average swallowing length, it is clarified that the number of frames indicating the swallowing length is small to large, and the swallowing length is in various situations and the number of swallowing blocks 34 is large, that is, the number of times of swallowing is large.

As described above, it is found that the number of frames included in one swallowing block 34 varies depending on the swallowing movement of the subject person in the entire examination video, and a distribution is different for each subject person. Therefore, it is considered that the feature values are effective as the feature values indicating the feature of the swallowing of the subject person by using the basic statistics such as the average value, the median value, the mode value, the variance, the standard deviation, the range, the maximum value, and the minimum value based on the number of frame images 31 included in each of the swallowing blocks 34 as the feature values, and thus the feature values are preferable as the feature values.

It is preferable that the feature value is the basic statistic based on the number of the frame images 31 in which the stage information is the initial swallowing-in-progress stage 32a in the frame images 31 included in the swallowing block 34. The whiteout frame image 31 in the initial swallowing-in-progress stage 32a is considered to be important among the images obtained in the video endoscopic examination of swallowing. Therefore, it is considered that calculating these basic statistics to use the basic statistics as the feature values is useful as the information for diagnosing the swallowing function.

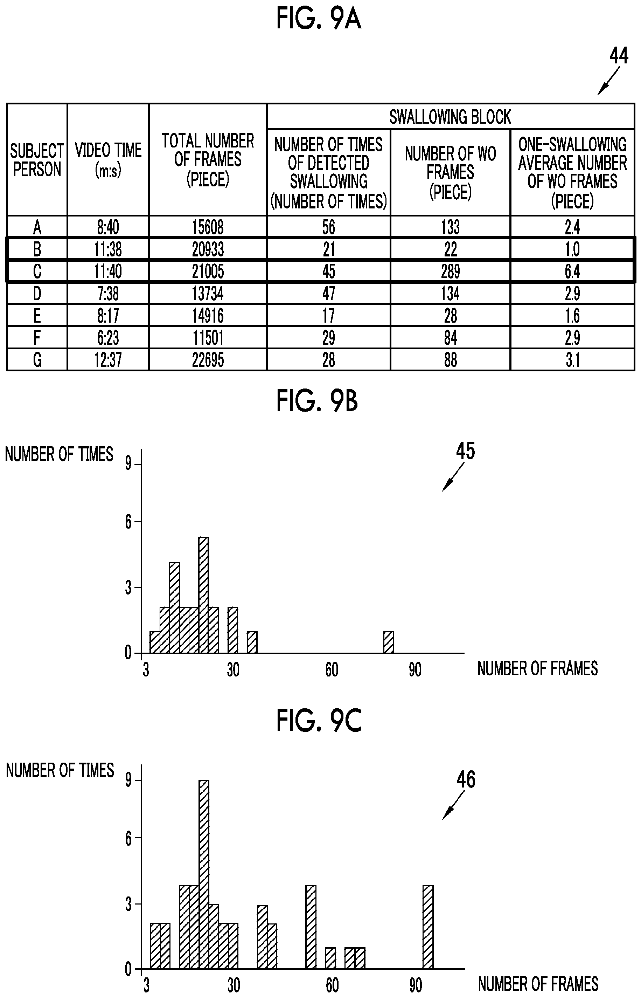

As shown in Fig. 9A, in the table, a video time (unit: min:sec) from the start of the examination to the end of the examination and the total number of frames (unit: piece) are summarized for the subject persons A to G who have undergone the video endoscopic examination of swallowing, and the number of times of detected swallowing (unit: number of times) and the total number of frames (unit: piece) which are included in each swallowing block 34 and in which the stage information is the initial swallowing-in-progress stage 32a and the average of the number of frames (unit: piece) which are included in each swallowing block 34 and in which the stage information is the initial swallowing-in-progress stage 32a as the basic statistics for the number of frames which are included in the each swallowing block 34 and in which the stage information is the initial swallowing-in-progress stage 32a are summarized for the swallowing block 34 detected within the video time. It should be noted that, in the table, the initial swallowing-in-progress stage 32a is described as whiteout (WO).

As shown in Table 44, in the swallowing block 34, in a case of focusing on the average ("one-swallowing average number of WO frames") of the number of frames which are included in each swallowing block 34 and in which the stage information is the initial swallowing-in-progress stage 32a, it is found that there is a difference of 6 times or more in the numerical value depending on the subject person. Therefore, as shown in Fig. 9B, in a case in which a histogram 45 shows the number of frames in which the stage information is the initial swallowing-in-progress stage 32a (whiteout) for each swallowing block 34 of the subject person B having the smallest one-swallowing average number of WO frames, that is, one swallowing movement, it is clarified that a large number of times of swallowing in which the number of whiteout frames in one swallowing movement is relatively small are performed.

On the other hand, as shown in Fig. 9C, in a case in which a histogram 46 shows the number of frames in which the stage information is the initial swallowing-in-progress stage 32a for one swallowing movement of the subject person C having the largest one-swallowing average number of WO frames, it is clarified that swallowing in which the number of whiteout frames in one swallowing movement is relatively small, swallowing in which the number of whiteout frames is relatively medium, and swallowing in which the number of whiteout frames is relatively large are dispersed, and that the number of swallowing blocks 34 is large, that is, the number of times of swallowing is large.

As described above, it is found that the number of whiteout frames included in one swallowing block 34 varies depending on the swallowing movement of the subject person in the entire examination video, and a distribution is different for each subject person. Therefore, it is considered that the feature values are effective as the feature values indicating the feature of the swallowing of the subject person by using the basic statistics such as the average value, the median value, the mode value, the variance, the standard deviation, the range, the maximum value, and the minimum value based on the number of whiteout frame images 31 included in each of the swallowing blocks 34 as the feature values, and thus the feature values are preferable as the feature values.

The feature value as described above is calculated as an objective value that suitably represents the feature of the swallowing regardless of the subjectivity of the doctor or the like who performs the examination, and thus is an objective indicator related to the swallowing. In addition, since these feature values are automatically calculated by the image processing device 15, the feature values can be generated without taking time and effort. These feature values can be used as one of the information for the diagnosis of the doctor, and the classification of the swallowing function and the like can be determined based on these feature values.

In addition, it is preferable that the feature value is calculated based on the lightness value of each of the plurality of consecutive frame images 31 captured in a specific period among the plurality of time-series frame images 31. It should be noted that the image acquisition unit 23 of the processor device 14 assigns the imaging time point information, which is a time point at which the frame image is captured, to the frame image 31. Therefore, the feature value calculation unit 27 can extract the frame image 31 in the specific period by using the imaging time point information with the frame image 31 in the swallowing stage, such as whiteout or blackout, as a starting point.

The specific period is preferably the plurality of consecutive frame images 31 captured in the specific period among the plurality of time-series frame images 31 included in the swallowing block 34. The specific period can be set to a first specific number of frame images 31 of the swallowing block 34, and for example, it is preferable to set the first three frame images 31 of the swallowing block 34.

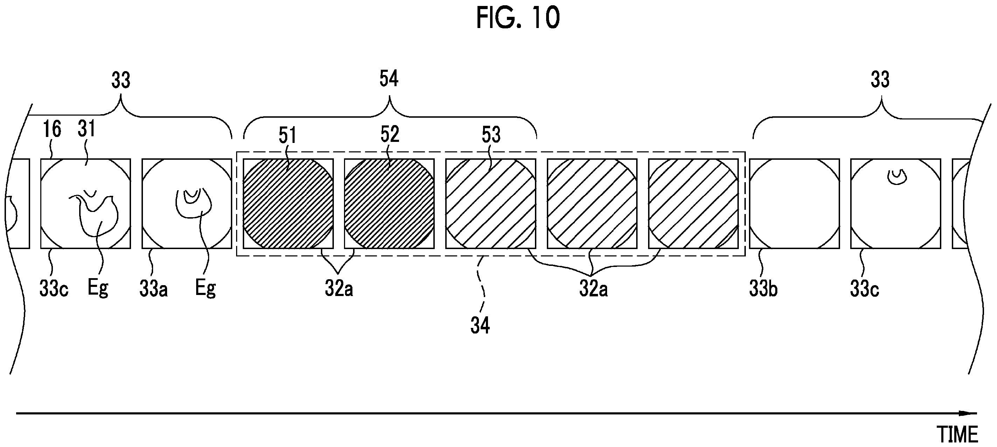

As shown in Fig. 10, it is preferable that the feature value calculation unit 27 is a basic statistic such as the average value calculated based on the lightness value of each frame image 31 for an image group 54 consisting of a frame image 51, a frame image 52, and a frame image 53 which are three consecutive frame images 31 among the plurality of time-series frame images 31 included in the swallowing block 34.

The first few consecutive frame images 31 of the swallowing block 34 include the whiteout frame image 31 in which the stage information is the initial swallowing-in-progress stage 32a. As described above, by using the lightness value of the frame image 31 in the initial swallowing-in-progress stage 32a as the feature value, it is possible to use an indicator such as the speed of the pharyngeal contraction, a contraction pressure of the pharyngeal contraction, and the contraction shape of the pharynx at the start of the swallowing, and for example, by using the average value of the lightness values of the image group 54 which is the first three frame images 31 of the swallowing block 34 as the feature value, it is possible to use these indicators after considering time.

In addition, it is preferable that the specific period is the plurality of frame images 31 captured in the specific period among the plurality of time-series frame images 31 that are not included in the swallowing block 34. The specific period can be a specific number of frame images 31 immediately after the swallowing block 34, and is, for example, preferably the first three frames immediately after the swallowing block 34. The first three frames immediately after the swallowing block 34 include the blackout frame image 31 in which the swallowing stage is the post-swallowing stage 33b.

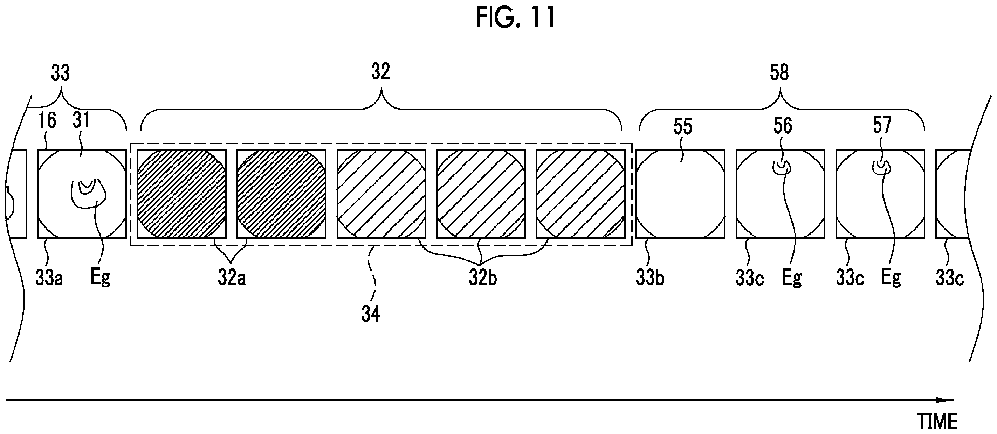

As shown in Fig. 11, it is preferable that the feature value calculation unit 27 is the basic statistic such as the average value calculated based on the lightness value of each frame image 31 for an image group 58 consisting of a frame image 55, a frame image 56, and a frame image 57 which are three frame images 31 immediately after the swallowing block 34.

A few frames immediately after the swallowing block 34 include the blackout frame image 31 in which the stage information is the post-swallowing stage 33b. As described above, by using the lightness value of the blackout frame image 31 as the feature value, it is possible to use an indicator such as an opening speed of the pharynx, an opening pressure of the pharynx, and an opening shape of the pharynx at the end of the swallowing, and for example, by using the average value of the lightness values of the image group 58 which is three frames immediately after the swallowing block 34 as the feature value, it is possible to use these indicators after considering the time.

As described above, the feature value can be set in accordance with what information is obtained as the information related to the swallowing. In a case in which the feature values considered as the feature values based on the frame image 31 included in the examination video are summarized, the following values can be used as the feature values. That is, examples of the feature value include the distribution of the plurality of swallowing blocks 34 themselves, the frequency of the frame image 31 in which the swallowing stage is the initial swallowing-in-progress stage 32a or the number of frame images 31, the area itself of the halation region in the frame image 31 in which the swallowing stage is the initial swallowing-in-progress stage 32a or a ratio of the area, the lightness value of the frame image 31 itself in which the swallowing stage is the initial swallowing-in-progress stage 32a, the frequency of the frame image 31 in which the swallowing stage is the post-swallowing stage 33b or the number of frame images 31, and the number of frame images 31 in which the swallowing stage included in each of the plurality of swallowing blocks 34 is the initial swallowing-in-progress stage 32a.

The lightness value in the feature value may be replaced with the blur amount. That is, the image acquisition unit 23 of the processor device 14 calculates the blur amount for the plurality of specific frame images 31. Therefore, it is preferable that the feature value is calculated based on the blur amount in the plurality of frame images 31 captured in the specific period among the plurality of time-series frame images 31. As the method of calculating the blur amount, a method of calculating the blur amount in the subject captured in the image in the related art can be used. The blur amounts in the plurality of frame images 31 are considered to indicate a speed of a movement of the observation target that is the subject. The speed of the movement of the pharyngeal contraction observed in the pre-swallowing stage is correlated with the blur amount. Therefore, it is considered that the feature value using the blur amount is a value indicating the feature of the swallowing.

As the feature value representing the speed of the movement of the pharyngeal contraction observed in the pre-swallowing stage, the magnitude of the image difference or the movement amount of the feature point may be used instead of the blur amount. Alternatively, the number of frames in which frames in which the movement amount of the feature point observed in the pre-swallowing stage is a certain threshold value appear consecutively may be used as the feature value. In the initial swallowing-in-progress stage, the movement amount of the feature point transitions from the swallowing-not-in-progress stage in which the movement amount of the feature point is small to the whiteout in which the movement amount of the feature point is small through the pharyngeal contraction phase in which the movement amount of the feature point is large. The number of frames in the pharyngeal contraction phase in which the movement amount of the feature point is large is considered as the feature value indicating the speed of the pharyngeal contraction.

Similar to the lightness value, for the blur amount, in addition to using the blur amount itself as the feature value, a basic statistic using the blur amount may be used as the feature value. For example, the maximum blur amount in the frame image in the specific period may be used as the feature value. In this case, as in the lightness value, the basic statistics can be used as the feature values for the blur amounts in the first three consecutive frame images included in the swallowing block 34 and the blur amount in the three consecutive frame images immediately after the swallowing block 34. It should be noted that a feature value obtained by combining both the lightness value and the blur amount may be calculated and used as a new feature value.

In addition, the examination video can be a video obtained by imaging the observation target in a case of the dry swallowing, a video obtained by imaging the observation target in a case of swallowing a certain amount of water, a video obtained by imaging the observation target in a case of swallowing a certain amount of swallowing food, and the like in the video endoscopic examination of swallowing. The dry swallowing can be swallowing in a case of swallowing saliva. In the video endoscopic examination of swallowing, the observation target in a case of swallowing saliva, water, various types of swallowing food, and the like is imaged. In the calculation of the feature value via the image processing device 15, the feature value related to the swallowing motion in these various situations can also be acquired.

Examples of the swallowing food include food in which a coloring agent or the like for more clearly visualizing a swallowing pathway or the above-described part is added, jelly-like food for checking the state of swallowing, food having various hardness, food having lumps of various sizes, and the like, in addition to water for observing the state of swallowing and the state of the mucous membrane.

It should be noted that, in the examination video in which the observation target in a case of swallowing water or various types of swallowing food is imaged, the recognition processing unit 26 may detect water or various types of swallowing food appearing in the frame image 31 included in the examination video. Even though the swallowing motion is completed, the degree of how long water, swallowing food, or the like remains in the frame image 31, the region in which the water, the swallowing food, or the like remains, the period in which the water, the swallowing food, or the like remains, and the like may be detected, and a value calculated regarding these factors may be used as the feature value.

It should be noted that the feature value calculation unit 27 may calculate different feature values in accordance with the type of the swallowing food to be swallowed. For example, the feature value of the entire examination video can be calculated for the examination video in a case of swallowing water, and the feature value in the swallowing block 34 can be calculated for the examination video in a case of swallowing the swallowing food.

In addition, the various feature values as described above may be used alone, or may be used as the feature value calculated by combining a plurality of feature values. In addition, a plurality of feature values may be calculated in parallel to use the plurality of feature values.

The storage unit 28 stores the examination video image used by the image processing device 15, stores the calculated feature value, or the like. The recognition processing unit 26, the feature value calculation unit 27, and the like can use the data stored in the storage unit 28.

The display controller 24 performs control of displaying the calculated feature value on the display 16, control of displaying the calculated feature value in a superimposed manner on the frame image 31, control of displaying various settings such as processing and functions of the image processing device 15, and the like. The display controller 24 performs control of displaying the feature value on the display 16 in accordance with the setting or the like of the user from the user interface 17.

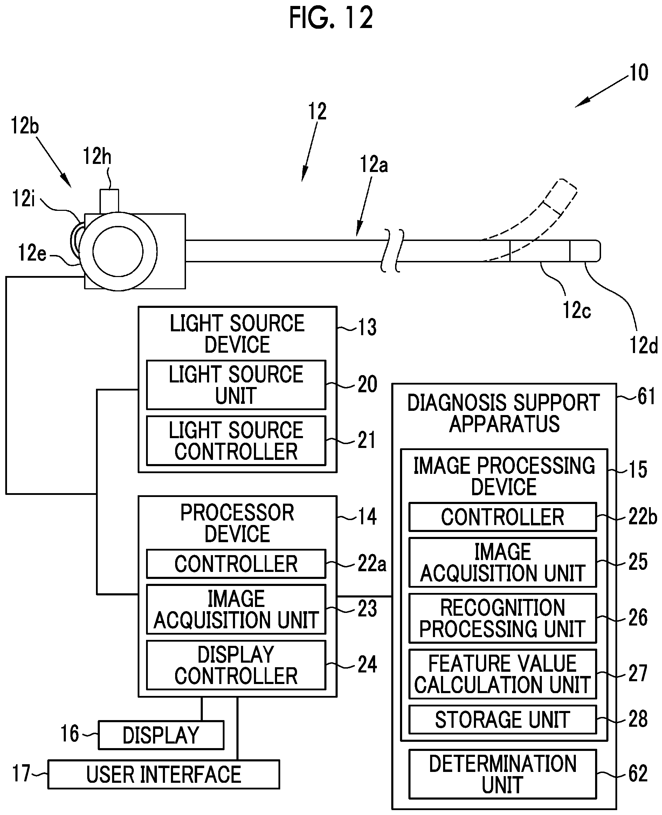

The feature value is used for the determination related to the swallowing function of the subject person. As shown in Fig. 12, the endoscope system 10 may comprise a diagnosis support apparatus 61. The diagnosis support apparatus 61 comprises the image processing device 15 and a determination unit 62. The determination unit 62 performs the determination related to the swallowing function of the subject person who has undergone the video endoscopic examination of swallowing based on the feature value calculated by the image processing device 15. The display controller 24 also performs display control in the diagnosis support apparatus 61. The display controller 24 performs control of displaying a result of the determination on the display 16.

The determination related to the swallowing function can be set in advance in accordance with the purpose, such as the determination of the feature of the swallowing and the determination of the swallowing function. Examples of the determination of the feature of the swallowing include a determination of a swallowing type. The distribution of the feature value, such as the distribution of the swallowing block 34, can be used for the determination of the swallowing type. The swallowing type can be classified into a type in which the swallowing movement is fast in both water and swallowing food, a type in which the swallowing movement is slow in both water and swallowing food, and the like.

In the determination of the swallowing function, the feature value using the examination video image in a case of swallowing water or various types of swallowing food can be used. The swallowing function determination can be classified, based on the feature value, into swallowing function good in which it is considered that the swallowing movement is appropriately performed in accordance with the swallowing food or the like, swallowing function possible in which it is considered that the swallowing movement is not appropriately performed due to the size, hardness, or the like of the swallowing food, and swallowing function impossible in which it is considered that a possibility of aspiration is high due to the size, hardness, or the like of the swallowing food.

In addition, the swallowing function may be determined in accordance with the swallowing food. The necessary feature value is calculated by using the examination video in a case in which each of three types of swallowing food of swallowing food A, swallowing food B, and swallowing food C having different types of hardness is swallowed. It should be noted that the swallowing food A is the softest swallowing food, the swallowing food B is the intermediate soft swallowing food, and the swallowing food C is the hardest swallowing food. With the determination of the swallowing function in a case of swallowing the swallowing food B as a reference, in a case of swallowing the swallowing food A, the determination of the swallowing function based on the feature value may be adjusted in a strict direction, and in a case of swallowing the swallowing food C, the determination of the swallowing function based on the feature value may be adjusted in a loose direction.

In addition, the determination unit 62 may determine whether or not to determine the swallowing function in accordance with the type of the swallowing food. In this case, the recognition processing unit 26 recognizes the type of the swallowing food based on the frame image 31 included in the examination video. This recognition can be automatically performed. In a case in which the swallowing food is recognized as the swallowing food A, the determination via the determination unit 62 may not be performed, and in a case in which the swallowing food is recognized as the swallowing food C, the determination via the determination unit 62 may be performed.

By automatically recognizing whether to perform the determination of the swallowing function in accordance with the swallowing food or to perform the determination of the swallowing function in accordance with the type of the swallowing food, and performing the determination of the swallowing function in a case of the specific swallowing food set in advance, the doctor or the like can obtain a certain determination result in the video endoscopic examination of swallowing without taking time and effort. These determination results are useful in various situations, and for example, it is possible to improve the efficiency of diagnosis, such as examining the examination video in the specific period without examining the entire examination video, and to continue the examination by determining the type of swallowing food based on the determination result automatically displayed during the video endoscopic examination of swallowing and to perform the examination content in one video endoscopic examination of swallowing more appropriately. Therefore, with the diagnosis support apparatus 61, it is possible to obtain useful information for supporting the diagnosis without taking time and effort.

It should be noted that the examination video captured from the start of the examination to the end of the examination is used as the examination video, but the user may designate the start point and the end point for calculating the feature value. The feature value may be calculated for the section defined based on the start point and the end point in the examination video.

In a case in which the processing performed by the image processing device 15 is desired to be performed on a plurality of parts in time series in one video endoscopic examination of swallowing, the plurality of parts may be set. In this case, the examination video includes a plurality of pairs of the start points and the end points. The examination video image without the swallowing food may be set by a start point 1 and an end point 1, the examination video image in a case of swallowing water may be set by a start point 2 and an end point 2, and the examination video image in a case of swallowing the swallowing food may be set by a start point 3 and an end point 3.

Examples of the designation method include a method of using various switches of the endoscope 12, a method of performing designation through the user interface 17, and a method of performing designation via voice recognition. In a case in which the switch of the endoscope 12 is used, for example, the still image acquisition instruction switch 12h may be used after being set as a trigger for designating the start point and the end point. In a case in which the end point is designated and the start point is not designated during the examination, the time point at which the still image acquisition instruction switch 12h is clicked may be designated as the start point, and the time point at which the still image acquisition instruction switch 12h is clicked again may be designated as the end time point of the examination.

As another designation method, the recognition processing unit 26 may receive designation of the start point and the end point via the user interface 17, and the feature value calculation unit 27 may calculate the feature value for the examination video image that is started from the start point and that ends at the first end point after the start point is started. Information on the imaging time point is assigned to the frame image 31 included in the examination video image. Therefore, the user can also select the part to be processed by the image processing device 15 by designating the start point and the end point of the video endoscopic examination of swallowing while displaying the examination video on the display 16.

The start point and the end point may be automatically set. The recognition processing unit 26 may set the frame image 31 in which the recognition result set in advance is obtained by the recognition of the frame image 31 as the start point, and may similarly set the frame image 31 in which the recognition result set in advance is obtained as the end point. As the recognition result set in advance, for example, the start of the swallowing block 34 may be set as the start point based on the stage information, and similarly, the whiteout frame image 31 in the post-swallowing stage 33b may be set as the end point. In addition, the recognition processing unit 26 may set, for the consecutive time-series frame images 31 in a case in which the specific swallowing food is recognized, a time point at which the frame image 31 having the earliest imaging time point is obtained as the start point, and a time point at which the frame image having the latest imaging time point is obtained as the end point.

It should be noted that, in the calculation of the feature value, the swallowing block or the frame image in the specific swallowing stage or period is extracted for the examination video for each subject person, but a plurality of examination videos acquired for the same subject person in different times may be analyzed. In a case in which the feature value is calculated for each of the examination videos acquired at a plurality of times before and after the swallowing function treatment of the same subject person, and the analysis, the diagnosis, and the like of the swallowing function and the like are performed based on the feature value, the case is preferable because the result can be used as an objective indicator indicating a change in the swallowing feature, an effect of the swallowing function treatment, a degree of progress of a decrease in the swallowing function, and the like.

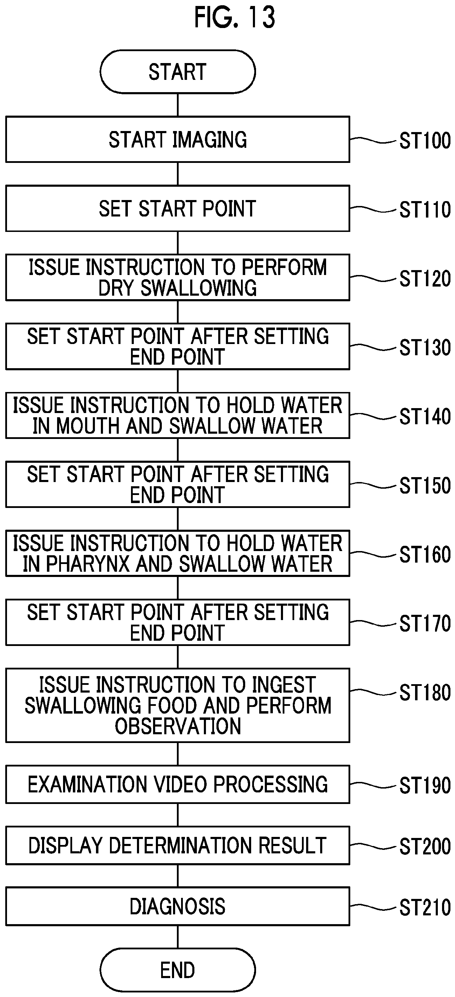

Next, an example of flows of the video endoscopic examination of swallowing and the diagnosis, and flows of processing in the image processing device 15 and the diagnosis support apparatus 61 will be described with reference to a flowchart. As shown in Fig. 13, in the video endoscopic examination of swallowing, the insertion part 12a of the endoscope 12 is inserted into the subject person, and the video capturing is started (step ST100). The distal end part 12d of the endoscope is disposed at an appropriate position, and four examinations of the swallowing, including dry swallowing, holding water in the mouth and swallowing the water, putting water in the pharynx and swallowing the water, and eating the swallowing food, are performed in order.

In each examination, at the start and end of the examination, the start point and the end point of the feature point calculation are indicated by clicking the still image acquisition instruction switch 12h of the endoscope 12. The still image acquisition instruction switch 12h has a function of an alternate switch that repeatedly provides an indication of the start point of the feature point calculation and an indication of the end point thereof each time the switch is clicked. In a case in which the still image acquisition instruction switch 12h of the endoscope 12 is first clicked, a mark serving as a mark of the start point of the feature value calculation is displayed on the display 16 along with the examination image. Then, the mark displayed on the display 16 disappears by clicking the still image acquisition instruction switch 12h. As a result, the user, such as the doctor, can set a period for calculating the feature value and can recognize this period.

By clicking the still image acquisition instruction switch 12h, the start point of the feature value is set (step ST110), and a signal for the subject person to perform the dry swallowing a plurality of times is issued (step ST120). Then, after the end point of the feature value is set, the start point of the feature value is set again (step ST130). A signal for the subject person to hold a certain amount (for example, 20 cc) of water in the mouth and swallow the water at once is issued (step ST140). Then, after the end point of the feature value is set, the start point of the feature value is set again (step ST150).

A signal for the subject person to hold a certain amount (for example, 20 cc) of water in the pharynx and swallow the water is issued (step ST160). Then, after the end point of the feature value is set, the start point of the feature value is set again (step ST170). An instruction to ingest the specific swallowing food is issued to the subject person, and the observation is performed (step ST180). After the observation is completed, the imaging ends, and the obtained examination video is analyzed (calculation and determination of the feature value) by the image processing device 15 and the diagnosis support apparatus 61 (step ST190). The determination result is displayed on the display 16 (step ST200). The doctor performs the diagnosis such as optimizing the rehabilitation program of the subject person based on the determination result obtained by the diagnosis support apparatus 61, which is the determination result displayed on the display 16 (step ST210).

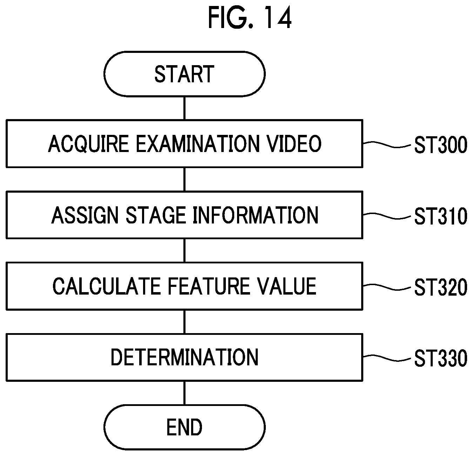

As shown in Fig. 14, as the processing (step ST190) performed by the image processing device 15 and the diagnosis support apparatus 61, first, the image acquisition unit 25 acquires the examination video acquired in the video endoscopic examination of swallowing, which is the examination video to which the start point and the end point of the feature value are assigned (step ST300). The recognition processing unit 26 performs the recognition processing for each frame image in the examination video image in the section for which the feature value is calculated, based on the start point and the end point of the feature value, and assigns the stage information (step ST310).

The feature value calculation unit 27 calculates the feature value designated in advance by using the frame image to which the stage information is assigned (step ST320). The determination unit 62 performs the determination related to the swallowing function based on the calculated feature value (step ST330). Thereafter, the processing is performed in the same manner as in step ST200 and subsequent steps, the display controller 24 displays the determination result on the display 16, and the doctor performs the diagnosis such as optimizing the rehabilitation program of the subject person based on the determination result displayed on the display 16.

It should be noted that the image processing device 15 or the diagnosis support apparatus 61 may be configured by a different computer from the processor device 14 and the image processing device 15 or the diagnosis support apparatus 61, or the processor device 14 may be configured to also exert the functions of the image processing device 15 and the diagnosis support apparatus 61. The image processing device 15 or the diagnosis support apparatus 61 may be disposed close to the processor device 14 or the like, or the image processing device 15 or the diagnosis support apparatus 61 may be disposed at a place away from the processor device 14 by communicating through a network.

In the present embodiment, the hardware structures (processor for the processor device, the control processor, and the like) of the processing units that perform various types of processing, such as the controller 22a, the controller 22b, the image acquisition unit 23, the display controller 24, the image acquisition unit 25, the recognition processing unit 26, the feature value calculation unit 27, and the determination unit 62, are various processors as shown below. The various processors include a CPU that is a general-purpose processor that executes software (programs) to function as various processing units, a graphics processing unit (GPU) that execute image processing at high speed, a programmable logic device (PLD) that is a processor capable of changing a circuit configuration after manufacture, such as a field programmable gate array (FPGA), and an exclusive electric circuit that is a processor having a circuit configuration exclusively designed to execute various types of processing.

One processing unit may be configured by one of these various processors, or may be configured by a combination of two or more same type or different type of processors (for example, a plurality of FPGAs, or a combination of a CPU and an FPGA). Also, a plurality of the processing units may be configured by one processor. As an example in which the plurality of processing units are configured by one processor, first, there is a form in which one processor is configured by a combination of one or more CPUs and software, and this processor functions as the plurality of processing units, as represented by a computer, such as a client or a server. Second, as represented by a system-on-chip (SoC) or the like, there is a form in which the processor is used in which the functions of the entire system which includes the plurality of processing units are realized by a single integrated circuit (IC) chip. In this way, various processing units are configured by one or more of the various processors described above, as the hardware structure.

Further, the hardware structure of these various processors is, more specifically, an electric circuit (circuitry) in a form of a combination of circuit elements, such as semiconductor elements. The hardware structure of a storage unit is a storage device, such as a hard disc drive (HDD) or a solid-state drive (SSD).

### Explanation of References

10: endoscope system
12: endoscope
12a: insertion part
12b: operating part
12c: bendable part
12d: distal end part
12e: angle knob
12h: still image acquisition instruction switch
12i: zoom operation unit
13: light source device
14: processor device
15: image processing device
16: display
17: user interface
20: light source unit
21: light source controller
22a, 22b: controller
23, 25: image acquisition unit
24: display controller
26: recognition processing unit
27: feature value calculation unit
28: storage unit
29: head
31: frame image
32: swallowing-in-progress stage
32a: initial swallowing-in-progress stage
32b: late swallowing-in-progress stage
33: swallowing-not-in-progress stage
33a: pre-swallowing stage
33b: post-swallowing stage
33c: open stage
34: swallowing block
41, 44: table
42, 43, 45, 46: histogram
51, 52, 53, 55, 56, 57: frame image
54, 58: image group
61: diagnosis support apparatus
62: determination unit
Es: esophagus
Eg: epiglottis
F: food
Rg: rima glottidis
Ps: pyriform sinus
Sp: soft palate
To: tongue
Tr: trachea
ST100 to ST210: step
ST300 to ST330: step

## Claims

1. An image processing device comprising:
a control processor,
wherein the control processor is configured to:
acquire an examination video capturing an observation target during a video endoscopic examination of swallowing;
perform recognition processing on the examination video for each frame image, to assign stage information indicating that an image shows which stage of the observation target among a plurality of predetermined swallowing stages to each of the frame images; and
calculate a feature value indicating a feature related to swallowing of a subject person having the observation target based on the stage information assigned to each of a plurality of the frame images included in the examination video.

2. The image processing device according to claim 1,
wherein the stage information includes a swallowing-in-progress stage indicating that the observation target is swallowing and a swallowing-not-in-progress stage indicating that the observation target is not swallowing, and
the swallowing-in-progress stage includes an initial swallowing-in-progress stage indicating that the observation target is in an initial stage of the swallowing that is in progress and a late swallowing-in-progress stage indicating that the observation target is in a late stage of the swallowing that is in progress.

3. The image processing device according to claim 2,
wherein the control processor is configured to detect a swallowing block that is a group of the frame images capturing a single swallowing motion of the observation target, and the swallowing block includes a plurality of consecutive frame images in which the stage information is the swallowing-in-progress stage.

4. The image processing device according to claim 3,
wherein the feature value is the number of the swallowing blocks.

5. The image processing device according to claim 3,
wherein the feature value is a basic statistic based on the number of the frame images included in each of the swallowing blocks.

6. The image processing device according to claim 2,
wherein the feature value is the number of the frame images in which the stage information is the initial swallowing-in-progress stage.

7. The image processing device according to claim 3,
wherein the feature value is a basic statistic based on the number of the frame images in which the stage information is the initial swallowing-in-progress stage in the frame images included in the swallowing block.

8. The image processing device according to claim 2,
wherein the swallowing-not-in-progress stage includes a post-swallowing stage in which the stage information of an immediately preceding frame image is the late swallowing-in-progress stage, and an open stage other than the post-swallowing stage.

9. The image processing device according to claim 8,
wherein the feature value is the number of the frame images in which the stage information is the post-swallowing stage.

10. The image processing device according to claim 2,
wherein the control processor is configured to calculate an area of a halation region in the frame image in which the stage information is the initial swallowing-in-progress stage, and
the feature value is the area of the halation region.

11. The image processing device according to claim 8,
wherein the control processor is configured to calculate a lightness value in the frame image in which the stage information is the post-swallowing stage, and
the feature value is the lightness value.

12. The image processing device according to claim 3,
wherein the control processor is configured to assign imaging time point information, that is a time point at which the frame image is captured, to the frame image, and
the feature value is calculated based on a lightness value of each of a plurality of consecutive frame images captured in a specific period, among a plurality of time-series frame images included in the swallowing block or among a plurality of time-series frame images not included in the swallowing block.

13. The image processing device according to claim 1,
wherein the examination video is obtained by imaging the observation target in a case in which a certain amount of water is swallowed in the video endoscopic examination of swallowing.

14. The image processing device according to claim 1,
wherein the examination video is obtained by imaging the observation target in a case in which a certain amount of swallowing food is swallowed in the video endoscopic examination of swallowing.

15. The image processing device according to claim 14,
wherein the control processor is configured to calculate a different feature value depending on a type of the swallowing food to be swallowed.

16. The image processing device according to claim 1,
wherein the control processor is configured to:
receive designation of a start point and an end point for calculating the feature value; and
calculate the feature value for a section defined based on the start point and the end point in the examination video.

17. The image processing device according to claim 1,
wherein the control processor is configured to perform control of displaying the feature value on a display.

18. An operation method of an image processing device, the operation method comprising:
acquiring an examination video capturing an observation target during a video endoscopic examination of swallowing;
performing recognition processing on the examination video for each frame image, to assign stage information indicating that an image shows which stage of the observation target among a plurality of predetermined swallowing stages to each of the frame images; and
calculating a feature value indicating a feature related to swallowing of a subject person having the observation target based on the stage information assigned to each of a plurality of the frame images included in the examination video.

19. A non-transitory computer readable medium for storing a computer-executable program, the computer-executable program causing a computer to implement:
a function of acquiring an examination video capturing an observation target during a video endoscopic examination of swallowing;
a function of performing recognition processing on the examination video for each frame image, to assign stage information indicating that an image shows which stage of the observation target among a plurality of predetermined swallowing stages to each of the frame images; and
a function of calculating a feature value indicating a feature related to swallowing of a subject person having the observation target based on the stage information assigned to a plurality of the frame images included in the examination video.

20. A diagnosis support apparatus comprising:
the image processing device according to any one of claims 1 to 17,
wherein the diagnosis support apparatus
performs a determination related to a swallowing function of the subject person who has undergone the video endoscopic examination of swallowing based on the feature value calculated by the image processing device, and
performs control of displaying the determination result on a display.
